(19) Europäisches Patentamt
European Patent Office
Office européen des brevets

(11) Veröffentlichungsnummer: **0 166 896**
**A1**

(12) # EUROPÄISCHE PATENTANMELDUNG

(21) Anmeldenummer: **85104953.6**

(22) Anmeldetag: **24.04.85**

(51) Int. Cl.[4]: **A 01 N 43/70**
C 07 D 251/50, C 07 D 251/52

(30) Priorität: **01.06.84 DE 3420490**

(43) Veröffentlichungstag der Anmeldung:
**08.01.86 Patentblatt 86/2**

(84) Benannte Vertragsstaaten:
**AT BE CH DE FR GB IT LI LU NL SE**

(71) Anmelder: **HÜLS AKTIENGESELLSCHAFT**
**- RSP Patente / PB 15 - Postfach 13 20**
**D-4370 Marl 1(DE)**

(72) Erfinder: **Schnurbusch, Horst, Dr.**
**Overwegstrasse 36**
**D-4690 Herne 1(DE)**

(72) Erfinder: **Baltruschat, Helmut, Dr.**
**Am Hagenbach 3**
**D-4405 Nottuln(DE)**

(54) **Verwendung von Acyl-s-triazinen zur selektiven Behandlung von Nutzpflanzen gegen Unkräuter und Schadgräser.**

(57) Die Erfindung betrifft die Verwendung von Acyl-s-triazinen der allgemeinen Formel

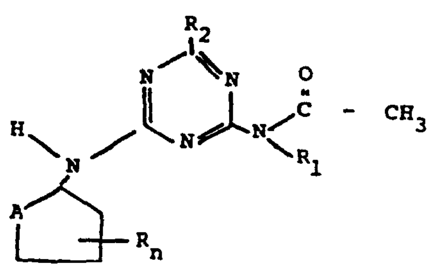

in der

$R_2$ Halogen, Alkoxy- oder Alkthio

$R_1$ einen gerad- oder verzweigtkettigen Alkyl-, Alkoxy-, Alkoxyalkyl- oder Alkylol-Rest,

R einen Rest aus der Gruppe der Alkyle mit ein bis drei C-Atomen und

A = $-CH_2-$ oder $-CH_2-CH_2-$ bedeuten und

n jeweils 0 oder 1, 2 oder 3

sein kann und wobei die Substituenten R an den Cycloalkylringen gleich oder verschieden sein können, in geeigneter Applikationsform zur selektiven Behandlung von Nutzpflanzen mit breitem Wirkungsspektrum gegen Unkräuter und Schadgräser vor und oder nach dem Auflaufen der Saat.

EP 0 166 896 A1

Croydon Printing Company Ltd

Verwendung von Acyl-s-triazinen zur selektiven Behandlung von Nutzpflanzen gegen Unkräuter und Schadgräser

Die Verwendung von symmetrischen Triazinen als Herbizid ist bekannt. Ursprünglich als Totalherbizid eingesetzt, liegt heute die Bedeutung dieser Substanzklasse mehr auf dem Gebiet der selektiven Anwendung. Die bekanntesten symmetrischen Triazine sind solche mit einem Cl-Atom, mit einem O-Alkylrest oder mit einem S-Alkylrest. Sie sind ausführlich in der Monographie von R. Wegler, "Chemie der Pflanzenschutz- und Schädlingsbekämpfungsmittel", Bd. 5, Springer-Verlag (1977), S. 336 ff, beschrieben. Bekannt sind z. B. auch die s-Triazine gemäß den DE-OSS 32 02 086, 32 22 147 und 33 22 720.

Des weiteren sind entsprechend der JP-OS 47 23 436 auch unsubstituierte 2-Cyclohexylamino-Derivate von s-Triazinen bekannt und gemäß DE-OS 32 22 147 auch 6-Methylthio-s-triazine, die mit alkylierten, cycloaliphatischen Aminen substituiert sind.

Bei diesen bekannten Wirkstoffen ist die Selektivität gegenüber Nutzpflanzen und die Herbizidwirkung gegen Schadpflanzen nicht voll befriedigend.

Überraschend wurde nun gefunden, daß bei den s-Triazinen die Verbindungen, die sowohl mit einem reinen oder gegebenenfalls alkylierten cycloaliphatischen Amin als auch mit einem acetylierten Amin substituiert sind, bei gleichzeitig guter herbizider Wirkung gegen Unkräuter eine sehr gute Selektivität in zahlreichen landwirtschaftlichen Kulturen zeigen.

Gegenstand der Erfindung ist also die Verwendung von Acyl-s-triazinen der allgemeinen Formel

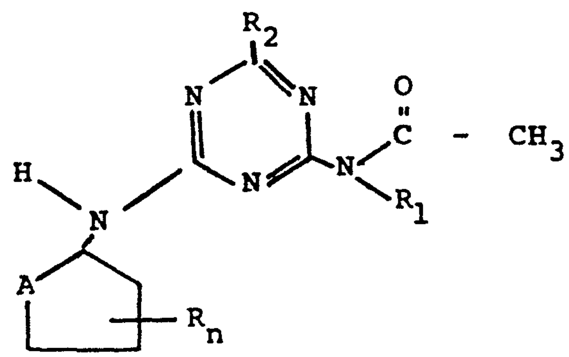

in der

$R_2$ Halogen, Alkoxy- oder Alkthio-

$R_1$ einen gerad- oder verzweigtkettigen Alkyl-, Alkoxy-, Alkoxyalkyl- oder Alkylol-Rest,

R einen Rest aus der Gruppe der Alkyle mit ein bis drei C-Atomen und

A = $-CH_2-$ oder $-CH_2-CH_2-$ bedeuten und

n jeweils 0 oder 1, 2 oder 3

sein kann und wobei die Substituenten R an den Cycloalkylringen gleich oder verschieden sein können, in geeigneter Applikationsform zur selektiven Behandlung von Nutzpflanzen mit breitem Wirkungsspektrum gegen Unkräuter und Schadgräser vor und/oder nach dem Auflaufen der Saat. Vorzugsweise erfolgt die Applikation vor Auflaufen der Saat.

Die Ketten, die von $R_1$ gebildet werden, sollten - bei Nichtberücksichtigung der H-Atome und unabhängig vom Verzweigungsgrad - nicht mehr als 15 C-(bzw. O)Atome aufweisen.

Bemerkenswert ist, daß bei gleichzeitig sehr guter herbizider Wirkung gegen Unkräuter und Schadgräser eine sehr gute Selektivität in zahlreichen landwirtschaftlichen Kulturen wie Reis (Oryza sativa), Gerste (Hordeum vulgare), Weizen (Triticum aestivum), Roggen (Secale secale), Soja (Glycine max) oder Baumwolle (Gossypium hirsutum) besteht.

Weiterhin besonders bemerkenswert bei dieser Stoffgruppe ist die sehr gute Wirkung gegen Hirsearten wie Echinochloa crus-galli (terrestrische und Sumpfvarietät), Echinochloa colonum, Digitaria sanguinalis und Setaria viridis. Bekanntlich sind bestimmte Hirsearten wie Echinochloa spec. wegen ihrer botanischen Verwandtschaft zu Reis in dieser Kultur besonders schwierig zu bekämpfen. Darüber hinaus besitzen die erfindungsgemäß eingesetzten Verbindungen überraschenderweise bei der Unkrautbekämpfung in Weizen, Gerste und Roggen eine hohe Selektivität bei gleichzeitig sehr guter Wirkung gegen Schadgräser, insbesondere Ackerfuchsschwanz und Windhalm. Sinngemäß das gleiche gilt für die Selektivität und herbizide Wirksamkeit der erfindungsgemäßen Verbindungen in Baumwolle, Mais und Sojabohnen. s-Triazine mit guter Wirkung gegen alle wichtigen Hirsearten sowie Ackerfuchsschwanz, Windhalm u. a. Schadgräser bei gleichzeitiger Selektivität in Getreidekulturen und besonders in Reis sind bisher nicht bekannt.

Die erfindungsgemäße Verwendung der Verbindungen stellt somit eine wertvolle Bereicherung der herbiziden Mittel zur selektiven Unkrautbekämpfung dar.

Die Herstellung der erfindungsgemäß verwendeten Triazine kann nach an sich bekannten Methoden erfolgen, indem man Cyanurchlorid - vorzugsweise in einem Lösungsmittel oder Lösungsmittelgemisch, z. B. in Aceton, Aceton/Wasser, Dioxan, Dioxan/Wasser, Tetrahydrofuran oder in Dimethylformamid - mit den entsprechenden Aminen bei der jeweils zweckmäßigen Temperatur, die gewöhnlich zwischen -30 °C und +50 °C liegt, umsetzt.

Zur selektiven Substitution am Triazingerüst arbeitet man nach der für die stufenweise Substitution in 1,3,5-Triazinen bekannten Arbeitsweise.

Die zuerst erhaltenen Chlor-Derivate werden in dem entsprechenden Alkohol oder anderen Lösungsmitteln mit Natriummethylat oder Natriumthiomethylat zu den entsprechenden Methoxy- oder Methylthio-alkylamino-s-triazinen umgesetzt.

Zu der nicht beanspruchten Herstellung werden zwei Beispiele angeführt.

<u>Herstellungsbeispiel 1</u>
Darstellung des 2-N-Acetyl-methylamino-4-cyclopentylamino-6-methoxy-s-triazin (Beispiel 89)

Ansatz:

| | | |
|---|---|---|
| 223 g | 2-Cyclopentylamino-4-methoxy-6-methylamino-s-triazin | 1,00 Mol |
| 81 g | Acetylchlorid | 1,03 Mol |
| 104 g | Triethylamin (TEA) | 1,03 Mol |
| 1 250 ml | Toluol, wasserfrei | |

Das s-Triazin wird zusammen mit dem TEA in 1 100 ml Toluol gelöst und auf 0 °C abgekühlt. Das Acetylchlorid wird in 150 ml Toluol gelöst und unter Rühren bei 0 - 5 °C in die Lösung des Ausgangsproduktes eingetropft. Die Eintropfdauer beträgt 30 min. Nachdem alles zugegeben ist, wird noch 30 min bei 0 - 5 °C gerührt und danach langsam unter Rühren auf 35 - 40 °C erwärmt. Der Ansatz wird noch 1 h

bei 40 °C gerührt. Der Niederschlag von TEA-Hydrochlorid wird dann abfiltriert und die ablaufende Lösung mit Wasser chloridfrei gewaschen. Beim Abdestillieren des Toluols fallen 227 g des acetylierten s-Triazins als weißes kristallines Produkt an. Die Ausbeute ist 85 %.

Herstellungsbeispiel 2

Darstellung des 2-N-Acetyl-methylamino-4-cyclopentylamino-6-methylthio-s-triazin (Beispiel 78)

Ansatz:

| | | |
|---|---|---|
| 239 g | 2-Cyclopentylamino-4-methylamino-6-methylthio-s-triazin | 1,0 Mol |
| 1 020 g | Essigsäureanhydrid | 10,0 Mol |

Der Ansatz wird unter Rühren aufgeheizt. Dabei geht das s-Triazin in Lösung. Nach Erreichen der Siedetemperatur von 143 °C wird 30 min am Rückfluß gekocht. Das entstandene Gemisch aus Essigsäure und Essigsäureanhydrid wird danach im Wasserstrahlvakuum abdestilliert. Nach scharfem Trocknen bei 80 °C im Ölvakuum fallen 25,3 g des acetylierten s-Triazins als fast weißes, festes Produkt an; das entspricht einer Ausbeute von ca. 90 % d. Th.

Tabelle 1a: Liste der hergestellten Acyl-s-triazine

Beispiel 1 = 2-N-Acetyl-methylamino-4-chloro-6-cyclopentylamino-s-triazin
Beispiel 2 = 2-N-Acetyl-ethylamino-4-chloro-6-cyclopentylamino-s-triazin
Beispiel 3 = 2-N-Acetyl-isopropylamino-4-chloro-6-cyclopentylamino-s-triazin
Beispiel 4 = 2-N-Acetyl-propylamino-4-chloro-6-cyclopentylamino-s-triazin
Beispiel 7 = 2-N-Acetyl-3'-ethoxy-propylamino-4-chloro-6-cyclopentylamino-s-triazin
Beispiel 8 = 2-N-Acetyl-3'-butoxy-propylamino-4-chloro-6-cyclopentylamino-s-triazin
Beispiel 9 = 2-N-Acetyl-3'-hexoxy-propylamino-4-chloro-6-cyclopentylamino-s-triazin
Beispiel 10 = 2-N-Acetyl-3'-isopropoxy-propylamino-4-chloro-6-cyclopentylamino-s-triazin
Beispiel 12 = 2-N-Acetyl-methylamino-4-chloro-6-cyclohexylamino-s-triazin
Beispiel 13 = 2-N-Acetyl-ethylamino-4-chloro-6-cyclohexylamino-s-triazin
Beispiel 14 = 2-N-Acetyl-isopropylamino-4-chloro-6-cyclohexylamino-s-triazin
Beispiel 15 = 2-N-Acetyl-propylamino-4-chloro-6-cyclohexylamino-s-triazin
Beispiel 23 = 2-N-Acetyl-methylamino-4-chloro-6-TMCP-amino-s-triazin
Beispiel 24 = 2-N-Acetyl-ethylamino-4-chloro-6-TMCP-amino-s-triazin
Beispiel 25 = 2-N-Acetyl-isopropylamino-4-chloro-6-TMCP-amino-s-triazin
Beispiel 26 = 2-N-Acetyl-propylamino-4-chloro-6-TMCP-amino-s-triazin
Beispiel 34 = 2-N-Acetyl-methylamino-4-chloro-6-TMC-amino-s-triazin

TMCP ≙ 2.2.4-(2.4.4)-Trimethylcyclopentyl
TMC ≙ 3.3.5-Trimethylcyclohexyl (cis/trans-Gemisch)

Beispiel 35 = 2-N-Acetyl-ethylamino-4-chloro-6-TMC-amino-s-triazin

Beispiel 36 = 2-N-Acetyl-isopropylamino-4-chloro-6-TMC-amino-s-triazin

Beispiel 37 = 2-N-Acetyl-propylamino-4-chloro-6-TMC-amino-s-triazin

Beispiel 57 = 2-N-Acetyl-ethylamino-4-methylthio-6-TMCP-amino-s-triazin

Beispiel 67 = 2-N-Acetyl-methylamino-4-cyclohexylamino-6-methylthio-s-triazin

Beispiel 68 = 2-N-Acetyl-ethylamino-4-cyclohexylamino-6-methylthio-s-triazin

Beispiel 69 = 2-N-Acetyl-isopropylamino-4-cyclohexylamino-6-methylthio-s-triazin

Beispiel 78 = 2-N-Acetyl-methylamino-4-cyclopentylamino-6-methylthio-s-triazin

Beispiel 79 = 2-N-Acetyl-ethylamino-4-cyclopentylamino-6-methylthio-s-triazin

Beispiel 80 = 2-N-Acetyl-isopropylamino-4-cyclopentylamino-6-methylthio-s-triazin

Beispiel 89 = 2-N-Acetyl-methylamino-4-cyclopentylamino-6-methoxy-s-triazin

Beispiel 90 = 2-N-Acetyl-ethylamino-4-cyclopentylamino-6-methoxy-s-triazin

Beispiel 101 = 2-N-Acetyl-ethylamino-4-methoxy-6-TMCP-amino-s-triazin

Beispiel 112 = 2-N-Acetyl-ethylamino-4-cyclohexylamino-6-methoxy-s-triazin

---

TMCP ≙ 2.2.4-(2.4.4)-Trimethylcyclopentyl

TMC ≙ 3.3.5-Trimethylcyclohexyl (cis/trans-Gemisch)

**Tabelle 1b:** Charakterisierung der hergestellten Acyl-s-triazine durch den Schmelzpunkt

| Beispiel | Schmelzpunkt (°C) |
|---|---|
| 1 | 98 |
| 2 | 30 (harzartig) |
| 3 | 75 |
| 4 | 75 |
| 7 | 30 (harzartig) |
| 8 | 30 (harzartig) |
| 9 | 30 (harzartig) |
| 10 | 30 (harzartig) |
| 12 | 155 |
| 13 | 105 |
| 14 | 70 |
| 15 | 138 |
| 23 | 125 |
| 24 | 80 |
| 25 | 50 |
| 26 | 30 (harzartig) |
| 34 | 30 (harzartig) |
| 35 | 30 (harzartig) |
| 36 | 30 (harzartig) |
| 37 | 85 |
| 57 | 30 (harzartig) |
| 67 | 129 |
| 68 | 85 |
| 69 | 56 |
| 78 | 94 |
| 79 | 51 |
| 80 | 30 (harzartig) |
| 89 | 30 (harzartig) |
| 90 | 30 (harzartig) |
| 101 | 30 (harzartig) |
| 112 | 30 (harzartig) |

Gegenüber anderen in der Literatur bekanntgewordenen Reisherbiziden wie Propachlor (N-Isopropyl-$\alpha$-Chloracetanilid) weisen die erfindungsgemäß eingesetzten Verbindungen den Vorteil der für s-Triazine bekannten Breitenwirkung sowohl gegen dikotyle als auch monokotyle Unkräuter auf.

Der Einsatz dieser Verbindungen ist nicht nur auf Kulturen der o. g. Pflanzenarten beschränkt, er kann auch erfolgreich in Kulturen anderer Pflanzen erfolgen.

In Abhängigkeit von der Konzentration können die Verbindungen auch zur Totalunkrautbekämpfung, z. B. auf Industrie- und Gleisanlagen oder auf Wegen und Plätzen mit und ohne Baumbewuchs, eingesetzt werden. Auch zur Unkrautbekämpfung in Dauerkulturen wie z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen sind die erfindungsgemäßen Verbindungen geeignet.

Bei einigen Pflanzenarten wirken einige der beanspruchten Verbindungen hemmend auf das Längenwachstum, ohne jedoch zu Ertragsminderungen zu führen; sie können deshalb auch zusätzlich als wachstumsregulierende Mittel eingesetzt werden. Manche der neuen Verbindungen können auch als Defoliants, Desiccants, Krautabtötungsmittel und Keimhemmungsmittel verwendet werden.

Applikation der Wirkstoffe

Die Wirkstoffe der erfindungsgemäßen Verbindungen können je nach Kultur in einer Menge von 0,1 bis 10 kg/ha, vorzugsweise von 0,5 bis 2,5 kg/ha, appliziert werden. Es können sowohl Racemate entsprechend einer Formel (Verbindung mit einem Wert für $R_1$, $R_2$ und R) als auch Gemische von erfindungsgemäßen Verbindungen (d. h. solche mit verschiedenen Bedeutungen für $R_1$ und/oder $R_2$ und/oder

R) zur Bekämpfung von Unkräutern und Ungräsern eingesetzt werden.

Außerdem ist es üblich, bei der Applikation ein oder mehrere Hilfsmittel aus der Gruppe der Träger-, Verdünnungs-, Lösungs-, Netz-, Haft-, Dispergier- und Emulgiermittel den Wirkstoffen zuzusetzen.

Auch die Zumischung anderer Herbizide bzw. der gleichzeitige Einsatz von Fungiziden, Insektiziden, Wachstumsregulatoren usw. mit den erfindungsgemäßen Wirkstoffen bzw. Wirkstoffgemischen ist möglich.

Schließlich können die erfindungsgemäßen Wirkstoffe oder Wirkstoffgemische, ggf. mit einem oder mehreren Vertretern aus den o. g. Stoffgruppen, Mineraldüngern oder Bodenverbesserungsmitteln zugesetzt und mit ihnen zusammen verteilt werden.

Im Vorauflauf erfolgt die Applikation vorzugsweise auf die Oberfläche vor dem Keimen der Samen und im Nachauflauf auf die Blattoberfläche im zweiten bis dritten Blattstadium.

Als Vergleichsmittel für die Gewächshausversuche dienten:

| | |
|---|---|
| Beispiel A: | 2-Chlor-4-(N-acetyl-isopropylamino)-6-(N-acetyl-ethylamino)-s-triazin |
| Beispiel B: | 2-Chlor-4-(N-isopropylamino)-6-(N-ethylamino)-s-triazin |
| Beispiel C: | S-(4-Chlorobenzyl)-N,N-diethylthiolcarbamat) |
| Beispiel D: | 2-Chlor-4-cyclopentylamino-6-methylamino-s-triazin |
| Beispiel E: | 2-Methylthio-4-cyclopentylamino-6-methylamino-s-triazin |

Gewächshaus-Versuchsreihe mit 4 kg/ha (Tabelle 3)
Für die meisten der erfindungsgemäßen Verbindungen wurden Gewächshaus-Versuche mit einer Aufwandmenge von 4 kg/ha, bezogen auf reinen Wirkstoff, an verschiedenen Testpflanzen durchgeführt.

Die jeweils benötigten Wirkstoffmengen wurden dabei in 1 000 l Wasser/ha suspendiert. Jeweils 3 Wochen nach der Behandlung der Kulturen mit dem Wirkstoff wurde bonitiert. Die Auswertung der Versuchsergebnisse erfolgte durch die übliche Benotung, bei der

1 = volle Wirkung = Abtötung der Pflanzen und
5 = keine Wirkung = Pflanzen wie unbehandelt

bedeuten.

Die in den Versuchen getesteten Pflanzenarten sind in Tabelle 2 aufgeführt:

Tabelle 2: Botanische Namen der Testpflanzen

| lateinische Bezeichnung | Code | deutsche Bezeichnung |
|---|---|---|
| Alopecurus myosuroides | ALOMY | Ackerfuchsschwanz |
| Amaranthus retroflexus | AMARE | Amaranth (zurückgekrümmter) |
| Apera spica-venti | APESV | Windhalm |
| Avena fatua | AVEFA | Flughafer |
| Avena sativa | AVESA | Hafer |
| Beta vulgaris | BETVU | Zuckerrübe |
| Brassica napus | BRANA | Winterraps |
| Digitaria sanguinalis | DIGSA | Blut-Fingerhirse |
| Echinochloa crus-galli | ECHCG | Hühnerhirse |
| Glycine max | GLYMA | Sojabohnen |
| Gossypium herbaceum | GOSHE | Baumwolle |
| Helianthus annuus | HELAN | Sonnenblume |
| Hordeum vulgare | HORVU | Gerste |
| Lamium purpureum | LAMPU | rote Taubnessel |
| Lycopersicum esculentum | LYCES | Tomate |

| lateinische Bezeichnung | Code | deutsche Bezeichnung |
|---|---|---|
| Matricaria spp. | MATSS | Kamille |
| Oryza sativa | ORYSA | Reis |
| Secale secale | SECSE | Roggen |
| Setaria viridis | SETVI | Grüne Borstenhirse |
| Sinapis arvensis | SINAR | Ackersenf |
| Stellaria media | STEME | Vogelmiere |
| Triticum aestivum | TRIAE | Weizen |
| Zea mays | ZEAMA | Mais |

Die erfindungsgemäß eingesetzten Verbindungen zeigen sowohl im Vor- als auch im Nachauflauf (Tabelle 3) eine hervorragende Wirkung gegen Schadgräser, wie z. B. Alopecurus myosuroides, Avena fatua und Echinochloa crus-galli und zweikeimblättrige Unkräuter, wie z. B. Sinapis arvensis.

<u>Gewächshaus-Versuchsreihe mit 3 und 2 kg/ha</u> (Tabelle 4)
Die gute herbizide Wirkung der erfindungsgemäßen Verbindungen wird auch bei einer Verringerung der Aufwandmenge von 3 und 2 kg, bezogen auf den reinen Wirkstoff, im Vor- und Nachauflauf bestätigt (Tabelle 4). Sowohl bei dikotylen Unkräutern wie Sinapis arvensis, Stellaria media und Matricaria spec. als auch bei Schadgräsern wie Echinochloa crus-galli, Avena fatua und Alopecurus ist die Wirkung der erfindungsgemäßen Verbindungen gut. Im Vergleich zur konstitutionell ähnlichen Verbindung A ist die Wirkung deutlich besser. Im Vergleich zum Standard B ist die Wirkung voll vergleichbar, jedoch mit dem Vorteil der guten Kulturverträglichkeit bei Reis, Soja und/oder Weizen.

<u>Gewächshaus-Versuchsreihe mit 1, 0,5 und 0,25 kg/ha</u>
(Tabelle 5)
Wegen der vorhandenen hohen herbiziden Wirkung der erfindungsgemäßen Verbindungen wurden einige zusätzlich in noch niedrigeren Aufwandmengen (1, 0,5 und 0,25 kg Wirkstoff/ha) im Vor- und Nachauflauf geprüft.

Die erfindungsgemäß eingesetzten Verbindungen zeigen ein weites Wirkungsspektrum und übertreffen in wichtigen Indikationen (u. a. Alopecurus myosuroides, Apera spica-venti, Avena fatua, Echinochloa crus-galli, Setaria viridis, Digitaria sanguinalis, Amaranthus retroflexus, Lamium purpureum, Matricaria spp.) die Wirkung der konstitutionell ähnlichen Verbindung A erheblich. Insbesondere die Wirkung gegen Hirsearten (Echinochloa, Setaria, Digitaria), die in verschiedenen Kulturen wie Reis, Baumwolle und Sojabohnen landwirtschaftlich wichtige Schadgräser darstellen, ist hervorzuheben. Die Wirkung gegen diese Hirsearten ist bei einer Reihe von erfindungsgemäß eingesetzten Verbindungen besser als beim Vergleichsprodukt B (Beispiele 78, 80, 89). Herausragend ist zugleich die gute Kulturverträglichkeit der erfindungsgemäßen Verbindungen bei Reis, Soja, Gerste, Roggen, Weizen und Mais. In den niedrigeren Aufwandmengen bei gleichzeitig guter herbizider Wirkung gegen Unkräuter zeigen einige erfindungsgemäße Verbindungen auch eine gute Kulturselektivität bei Zuckerrüben und Raps. Das Vergleichsmittel B zeigt nur hinreichende Kulturselektivität in Mais, nicht jedoch in den übrigen aufgeführten Kulturarten.

<u>Selektive herbizide Wirkung auf Reis</u> (Tabelle 6)
25 Tage alte Reissetzlinge wurden - jeweils doppelt zu einer Pflanze gesetzt - in große quadratische Eternitcontainer verpflanzt. Zwischen die Reihen der Reispflanzen wurden Samen der in Reis bedeutsamen Wildhirse Echinochloa crus-galli ("Watergrass", Sumpfvarietät) gesät. Die Schalen wurden gut bewässert und bei einer Temperatur von ca. 25 °C und 95 % rel. Luftfeuchtigkeit gehalten. Nach 10 Tagen, also im 2 - 3 Blattstadium der Wildhirse wurde die Erde in der Schale mit einer 2,5 cm hohen Schicht Wasser bedeckt. Der Wirkstoff wurde in einer geeigneten Formulierung (Emulsions-, Suspensionskonzentrat oder wettable Powder) mittels einer Pipette zwischen die

Pflanzenreihen appliziert, wobei der Wirkstoff so mit Wasser verdünnt war, daß die Applikationsmenge 3, 2, 1 und 0,5 kg a. i./ha entsprach. Der Versuch wurde nach 4 Wochen ausgewertet. Für die Bonitierung wurden die Frischgewichte der behandelten Pflanzen mit den Kontrollpflanzen in Beziehung gesetzt und die jeweiligen Wirkungsgrade in % wiedergegeben.

Aus Tabelle 6 ist ersichtlich, daß die erfindungsgemäßen Verbindungen dem Vergleichsmittel C, einem bekannten Reisherbizid, in der Wirkung gegen Echinochloa crus-galli (in der Varietät als "watergrass"), dem wichtigsten Schadgras in Reis, überlegen sind. Bei den Verbindungen 78 und 89 beträgt die Wirkung gegen diese Wildhirse selbst in der niedrigsten geprüften Wirkstoffmenge 95 bzw. 100 %, während bei dem Vergleichsmittel C bei dieser Aufwandmenge nur 61 % erreicht werden. Auch gegenüber den konstitutionell ebenbürtigen Vergleichsmitteln D und E bestehen insofern Vorteile, als die erfindungsgemäßen Verbindungen gegenüber dem Vergleichsmittel E eine bessere Wirkung gegen Echinochloa crus-galli aufweisen und die Vergleichsverbindung D in Reis nicht selektiv ist. Die Reis-Selektivität der erfindungsgemäß verwendeten Verbindungen ist gut, während das Vergleichsmittel B unter den beschriebenen Versuchsbedingungen keine Reis-Selektivität aufweist.

Tabelle 3:
Selektive herbizide Wirkung der erfindungsgemäßen Verbindungen vor dem Auflaufen (VA) bzw. nach dem Auflaufen (NA) der Testpflanzen

| Verbindung Nr. | kg Wirkstoff a.i./ha | SINAP | | LYCES | | ECHCG | | AVESA | | AVEFA | | ALOMY | |
|---|---|---|---|---|---|---|---|---|---|---|---|---|---|
| | | VA | NA | VA | NA | VA | NA | VA | NA | VA | NA | VA | NA |
| 1 | 4 | 2 | 1 | 1 | 1 | 1 | 5 | 1 | 2 | 1 | 1 | 1 | 1 |
| 2 | 4 | 1 | 1 | 1 | 1 | 2 | 3 | 3 | 2 | 1 | 1 | 1 | 1 |
| 3 | 4 | 1 | 2 | 1 | 1 | 1 | 1 | 1 | 4 | 1 | 1 | 1 | 1 |
| 4 | 4 | 2 | 3 | 1 | 1 | 4 | 5 | 3 | 4 | 1 | 2 | 1 | 1 |
| 12 | 4 | 1 | 1 | 1 | 1 | 5 | 5 | 5 | 5 | 3 | 3 | 1 | 5 |
| 13 | 4 | 1 | 1 | 1 | 1 | 5 | 4 | 4 | 5 | 1 | 1 | 1 | 1 |
| 14 | 4 | 5 | 3 | 5 | 1 | 5 | 5 | 4 | 3 | 3 | 4 | 1 | 2 |
| 15 | 4 | 5 | 2 | 1 | 1 | 3 | 5 | 3 | 4 | 1 | 1 | 1 | 3 |
| 24 | 4 | 5 | 5 | 4 | 1 | 5 | 5 | 5 | 5 | 5 | 4 | 5 | 5 |
| 35 | 4 | 5 | 1 | 2 | 1 | 3 | 2 | 2 | 1 | 2 | 1 | 1 | 1 |
| 57 | 4 | 5 | 1 | 1 | 1 | 1 | 1 | 4 | 1 | 1 | 1 | 1 | 1 |
| 67 | 4 | 1 | 1 | 1 | 1 | 1 | 1 | 1 | 1 | 1 | 1 | 1 | 1 |
| 68 | 4 | 1 | 1 | 1 | 1 | 1 | 1 | 1 | 1 | 1 | 1 | 1 | 1 |
| 69 | 4 | 1 | 1 | 1 | 1 | 1 | 1 | 1 | 1 | 1 | 1 | 1 | 1 |
| 78 | 4 | 1 | 1 | 1 | 1 | 1 | 1 | 1 | 1 | 1 | 1 | 1 | 1 |
| 79 | 4 | 1 | 1 | 1 | 1 | 1 | 1 | 1 | 1 | 1 | 1 | 1 | 1 |
| 80 | 4 | 1 | 1 | 1 | 1 | 1 | 1 | 1 | 1 | 1 | 1 | 1 | 1 |
| 89 | 4 | 1 | 1 | 1 | 1 | 1 | 1 | 1 | 1 | 1 | 1 | 1 | 1 |
| 90 | 4 | 1 | 1 | 1 | 1 | 1 | 1 | 1 | 1 | 1 | 1 | 1 | 1 |
| 91 | 4 | 1 | 1 | 1 | 1 | 1 | 1 | 1 | 1 | 1 | 1 | 1 | 1 |
| 92 | 4 | 1 | 1 | 1 | 1 | 1 | 1 | 1 | 1 | 1 | 1 | 1 | 1 |
| 101 | 4 | 1 | 1 | 1 | 1 | 1 | 1 | 1 | 1 | 1 | 1 | 1 | 1 |
| 111 | 4 | 1 | 1 | 1 | 1 | 1 | 1 | 1 | 1 | 1 | 1 | 1 | 1 |
| 112 | 4 | 1 | 1 | 1 | 1 | 1 | 1 | 1 | 1 | 1 | 1 | 1 | 1 |
| 113 | 4 | 1 | 1 | 1 | 1 | 1 | 1 | 1 | 1 | 1 | 1 | 1 | 1 |
| A (bekannt) | 4 | 1 | 1 | 1 | 1 | 2 | 2 | 2 | 2 | 1 | 1 | 1 | 1 |

Tabelle 4: Selektive herbizide Wirkung der erfindungsgemäßen Verbindungen in den Aufwandmengen 3 und 2 kg a.i./ha vor dem Auflaufen (VA) bzw. nach dem Auflaufen (NA) der Testpflanzen

| Verbindung Nr. | kg Wirkstoff a.i./ha | SINAR | | LYCES | | ECHGC | | TRIAE | | AVEFA | | ALOMY | | GLYMA | | STEME | | MATSS | | ORYSA | |
|---|---|---|---|---|---|---|---|---|---|---|---|---|---|---|---|---|---|---|---|---|---|
| | | VA | NA | VA | NA | VA | NA | VA | NA | VA | NA | VA | NA | VA | NA | VA | NA | VA | NA | VA | VA |
| A (bekannt) | 3<br>2 | 1<br>2 | 1<br>1 | 1<br>1 | 1<br>1 | 2<br>2 | 3<br>4 | 3<br>3 | 1<br>1 | 4<br>4 | 1<br>1 | 1<br>1 | 1<br>1 | 5 | 3<br>4 | 1<br>1 | 1<br>1 | 1<br>1 | 1<br>1 | 2<br>2 | 2<br>2 |
| B (bekannt) | 3<br>2 | 1<br>1 | 1<br>1 | 1<br>1 | 1<br>1 | 1<br>1 | 1<br>1 | 1<br>1 | 1<br>1 | 1<br>1 | 1<br>1 | 1<br>1 | 1<br>1 | 1<br>1 | 1<br>1 | 1<br>1 | 1<br>1 | 1<br>1 | 1<br>1 | 2<br>2 | 1<br>1 |
| 1 | 3<br>2 | 2<br>1 | 1<br>1 | 1<br>1 | 1<br>1 | 1<br>1 | 5 | 3<br>5 | 2<br>5 | 1<br>1 | 1<br>1 | 1<br>1 | 1<br>1 | 2<br>3 | 2<br>3 | 1<br>1 | 1<br>1 | 1<br>1 | 1<br>1 | 2<br>3 | 2<br>3 |
| 2 | 3<br>2 | 1<br>2 | 1<br>1 | 1<br>1 | 1<br>1 | 2<br>2 | 3<br>3 | 3<br>5 | 3<br>5 | 2<br>3 | 1<br>1 | 1<br>1 | 1<br>1 | 3<br>5 | 2<br>4 | 1<br>1 | 1<br>1 | 1<br>1 | 1<br>1 | 2<br>3 | 2<br>3 |
| 3 | 3<br>2 | 1<br>1 | 2<br>2 | 1<br>1 | 1<br>1 | 1<br>1 | 1<br>1 | 3<br>4 | 3<br>5 | 1<br>1 | 1<br>1 | 1<br>1 | 1<br>1 | 4<br>2 | 2<br>5 | 1<br>1 | 1<br>1 | 1<br>1 | 1<br>1 | 2<br>4 | 3<br>3 |
| 4 | 3<br>2 | 1<br>2 | 1<br>3 | 1<br>1 | 1<br>1 | 5 | 5 | 5 | 5 | 3<br>4 | 1<br>2 | 3<br>3 | 3<br>4 | 5 | 5 | 1<br>1 | 1<br>1 | 1<br>1 | 1<br>1 | 2<br>4 | 2<br>3 |
| 12 | 3<br>2 | 1<br>2 | 1<br>3 | 1<br>1 | 1<br>1 | 5 | 5 | 5 | 5 | 4<br>4 | 3<br>3 | 3<br>3 | 2<br>2 | 5 | 5 | 1<br>1 | 1<br>1 | 1<br>1 | 3<br>5 | 4<br>4 | 4<br>5 |
| 13 | 3<br>2 | 1<br>4 | 1<br>1 | 1<br>1 | 1<br>1 | 5 | 4<br>5 | 5 | 5 | 4<br>4 | 1<br>1 | 1<br>1 | 1<br>1 | 5 | 4<br>5 | 1<br>1 | 1<br>1 | 1<br>1 | 3<br>3 | 2<br>4 | 2<br>5 |
| 57 | 3<br>2 | 5<br>5 | 1<br>1 | 1<br>1 | 1<br>1 | 1<br>1 | 1<br>1 | 5 | 5 | 1<br>2 | 1<br>1 | 1<br>1 | 1<br>1 | 5<br>5 | 3<br>3 | 1<br>1 | 1<br>1 | 1<br>1 | 1<br>1 | 5 | 5 |
| 68 | 3<br>2 | 1<br>5 | 1<br>1 | 1<br>1 | 1<br>1 | 1<br>1 | 1<br>1 | 1<br>1 | 4<br>5 | 1<br>1 | 1<br>1 | 1<br>1 | 1<br>1 | 3<br>5 | 1<br>3 | 1<br>1 | 1<br>1 | 1<br>1 | 1<br>1 | 5 | 5 |
| 78 | 3<br>2 | 1<br>1 | 1<br>1 | 1<br>1 | 1<br>1 | 1<br>1 | 1<br>1 | 1<br>1 | 1<br>1 | 1<br>1 | 1<br>1 | 1<br>1 | 1<br>1 | 1<br>2 | 1<br>1 | 1<br>1 | 1<br>1 | 1<br>1 | 1<br>1 | 5 | 5 |

Tabelle 4 (Fortsetzung)

Selektive herbizide Wirkung der erfindungsgemäßen Verbindungen in den Aufwandmengen 3 und 2 kg a.i./ha vor dem Auflaufen (VA) bzw. nach dem Auflaufen (NA) der Testpflanzen

| Verbindung Nr. | kg Wirkstoff a.i./ha | SINAP VA | SINAP NA | LYCES VA | LYCES NA | ECHCG VA | ECHCG NA | TRIAE VA | TRIAE NA | AVEFA VA | AVEFA NA | ALOMY VA | ALOMY NA | GLYMA VA | GLYMA NA | STEME VA | STEME NA | MATSS VA | MATSS NA | ORYSA VA | ORYSA NA |
|---|---|---|---|---|---|---|---|---|---|---|---|---|---|---|---|---|---|---|---|---|---|
| 79 | 3 | 1 | 1 | 1 | 1 | 1 | 1 | 1 | 1 | 1 | 1 | 1 | 1 | 1 | 1 | 1 | 1 | 1 | 1 | 5 | 5 |
| | 2 | 1 | 1 | 1 | 1 | 1 | 1 | 1 | 1 | 1 | 1 | 1 | 1 | 2 | 1 | 1 | 1 | 1 | 1 | | |
| 80 | 3 | 1 | 1 | 1 | 1 | 1 | 1 | 1 | 1 | 1 | 1 | 1 | 1 | 3 | 1 | 1 | 1 | 1 | 1 | 5 | 5 |
| | 2 | 1 | 1 | 1 | 1 | 1 | 1 | 3 | 4 | 1 | 1 | 1 | 1 | 4 | 1 | 1 | 1 | 1 | 1 | | |
| 89 | 3 | 1 | 1 | 1 | 1 | 1 | 1 | 2 | 1 | 1 | 1 | 1 | 1 | 1 | 1 | 1 | 1 | 1 | 1 | 5 | 5 |
| | 2 | 1 | 1 | 1 | 1 | 1 | 1 | 2 | 1 | 1 | 1 | 1 | 1 | 1 | 1 | 1 | 1 | 1 | 1 | | |
| 90 | 3 | 1 | 1 | 1 | 1 | 1 | 1 | 1 | 1 | 1 | 1 | 1 | 1 | 1 | 1 | 1 | 1 | 1 | 1 | 5 | 5 |
| | 2 | 1 | 1 | 1 | 1 | 1 | 1 | 2 | 2 | 1 | 1 | 1 | 1 | 1 | 1 | 1 | 1 | 1 | 1 | | |
| 91 | 3 | 1 | 1 | 1 | 1 | 1 | 1 | 2 | 1 | 1 | 1 | 1 | 1 | 1 | 1 | 1 | 1 | 1 | 1 | 5 | 5 |
| | 2 | 1 | 1 | 1 | 1 | 1 | 1 | 4 | 1 | 1 | 1 | 1 | 1 | 1 | 1 | 1 | 1 | 1 | 1 | | |
| 101 | 3 | 1 | 1 | 1 | 1 | 2 | 2 | 5 | 5 | 5 | 1 | 1 | 1 | 5 | 1 | 1 | 1 | 1 | 1 | 5 | 5 |
| | 2 | 1 | 1 | 1 | 1 | 3 | 4 | | | | | 1 | 1 | | 1 | 1 | 1 | 1 | 1 | | |
| 112 | 3 | 1 | 1 | 1 | 1 | 1 | 1 | 4 | 1 | 1 | 1 | 1 | 1 | 2 | 1 | 1 | 1 | 1 | 1 | 5 | 5 |
| | 2 | 1 | 1 | 1 | 1 | 1 | 1 | 4 | 1 | 1 | 1 | 1 | 1 | 3 | 1 | 1 | 1 | 1 | 1 | | |

Tabelle 5:

Selektive herbizide Wirkung der erfindungsgemäßen Verbindungen in den Aufwandmengen 1, 0,5 und 0,25 kg a.i./ha vor dem Auflaufen (VA) bzw. nach dem Auflaufen (NA) der Testpflanzen

| Verbindung Nr. | kg Wirkstoff a.i./ha | ALOMY | | AMARE | | APESV | | AVEFA | | AVESA | | BETVU | | BRANA | | DIGSA | | ECHCG | | GLYMA | | GOSHI | |
|---|---|---|---|---|---|---|---|---|---|---|---|---|---|---|---|---|---|---|---|---|---|---|---|
| | | VA | NA | VA | NA | VA | NA | VA | NA | VA | NA | VA | NA | VA | NA | VA | NA | VA | NA | VA | NA | VA | NA |
| A (bekannt) | 1<br>0,5<br>0,25 | 1<br>2<br>3 | 3<br>3<br>4 | 1<br>3<br>3 | 1<br>2<br>3 | 1<br>2<br>3 | 1<br>2<br>5 | 4<br>4<br>5 | 4<br>4<br>5 | 4<br>4<br>5 | 3<br>3<br>5 | 2<br>4<br>5 | 2<br>3<br>3 | 4<br>4<br>5 | 5 | 3<br>5 | 5 | 3<br>5 | 5 | 5 | 5 | 5 | 5 |
| B (bekannt) | 1<br>0,5<br>0,25 | 1<br>1<br>2 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>2<br>4 | 3<br>5 | 1<br>1<br>2 | 1<br>1<br>1 | 1<br>1<br>1 | 1<br>1<br>3 | 2<br>4<br>5 | 2<br>3<br>5 |
| 68 | 1<br>0,5<br>0,25 | 1<br>1<br>2 | 1<br>1<br>2 | 1<br>3<br>3 | 1<br>2<br>2 | 1<br>1<br>2 | 1<br>1<br>3 | 3<br>4<br>5 | 2<br>3<br>5 | 1<br>1<br>2 | 1<br>2<br>3 | 1<br>1<br>2 | 1<br>1<br>4 | 5 | 1<br>1<br>3 | 1<br>1<br>3 | 1<br>2<br>3 | 1<br>1<br>3 | 1<br>2<br>4 | 5 | 3<br>3<br>5 | 3<br>4<br>5 | 3<br>5 |
| 78 | 1<br>0,5<br>0,25 | 1<br>1<br>1 | 1<br>1<br>3 | 1<br>1<br>4 | 1<br>1<br>2 | 1<br>1<br>1 | 1<br>1<br>1 | 1<br>1<br>2 | 1<br>1<br>3 | 1<br>3<br>3 | 3<br>3<br>4 | 2<br>2<br>3 | 1<br>1<br>2 | 4<br>5 | 1<br>1<br>1 | 1<br>2<br>2 | 1<br>1<br>1 | 1<br>1<br>1 | 1<br>1<br>1 | 1<br>3<br>3 | 2<br>3<br>4 | 3<br>3<br>5 | 3<br>5 |
| 80 | 1<br>0,5<br>0,25 | 1<br>2<br>3 | 1<br>2<br>4 | 1<br>5 | 1<br>2<br>2 | 1<br>2<br>3 | 1<br>2<br>3 | 1<br>3<br>3 | 1<br>1<br>4 | 2<br>3<br>3 | 1<br>4<br>4 | 1<br>2<br>3 | 1<br>1<br>2 | 5 | 1<br>1<br>2 | 1<br>2<br>2 | 1<br>1<br>1 | 1<br>1<br>3 | 1<br>1<br>5 | 4<br>5 | 4<br>5 | 3<br>5 | 4<br>5 |
| 89 | 1<br>0,5<br>0,25 | 1<br>1<br>2 | 1<br>1<br>2 | 1<br>1<br>1 | 1<br>1<br>1 | 1<br>1<br>1 | 1<br>1<br>1 | 1<br>1<br>3 | 1<br>1<br>3 | 1<br>2<br>2 | 2<br>4<br>5 | 1<br>3<br>3 | 1<br>1<br>1 | 3<br>3<br>4 | 1<br>1<br>2 | 1<br>1<br>2 | 1<br>1<br>2 | 1<br>1<br>1 | 1<br>1<br>1 | 3<br>4<br>4 | 1<br>1<br>2 | 3<br>5 | 4<br>5 |
| 112 | 1<br>0,5<br>0,25 | 2<br>2<br>4 | 2<br>2<br>2 | 1<br>5<br>5 | 1<br>1<br>1 | 2<br>2<br>4 | 2<br>2<br>2 | 1<br>4<br>4 | 1<br>1<br>2 | 2<br>4<br>5 | 2<br>2<br>2 | 1<br>4<br>5 | 1<br>1<br>1 | 2<br>4<br>5 | 1<br>1<br>3 | 1<br>2<br>4 | 1<br>1<br>2 | 1<br>2<br>4 | 1<br>1<br>2 | 5 | 1<br>1<br>1 | 3<br>4<br>5 | 4<br>5 |

Tabelle 5: (Fortsetzung)

Selektive herbizide Wirkung der erfindungsgemäßen Verbindungen in den Aufwandmengen 1, 0,5 und 0,25 kg a.i./ha vor dem Auflaufen (VA) bzw. nach dem Auflaufen (NA) der Testpflanzen

| Verbindung Nr. | kg Wirkstoff a.i./ha | HORVU | | LAMPU | | LYCES | | MATSS | | ORYSA | | SECSE | | SETVI | | SINAP | | STEME | | TRIAE | | ZEAMA | |
|---|---|---|---|---|---|---|---|---|---|---|---|---|---|---|---|---|---|---|---|---|---|---|---|
| | | VA | NA | VA | NA | VA | NA | VA | NA | VA | NA | VA | NA | VA | NA | VA | NA | VA | NA | VA | NA | VA | NA |
| A (bekannt) | 1<br>0,5<br>0,25 | 2<br>5 | 3<br>5 | 1<br>3<br>5 | 1<br>4<br>5 | 1<br>2<br>5 | 1<br>1<br>3 | 1<br>1<br>1 | 5 | 3<br>4<br>5 | 2<br>3<br>5 | 2<br>4<br>5 | 3<br>5 | 3<br>5 | 5 | 3<br>4<br>5 | 2<br>5 | 1<br>5 | 3<br>3<br>5 | 3<br>5 | 4<br>5 | 5 | 5 |
| B (bekannt) | 1<br>0,5<br>0,25 | 1<br>1<br>3 | 1<br>1<br>3 | 1<br>1<br>1 | 1<br>1<br>1 | 1<br>1<br>1 | 1<br>1<br>1 | 1<br>1<br>1 | 1<br>1<br>1 | 1<br>1<br>3 | 1<br>1<br>2 | 2<br>3<br>3 | 1<br>3<br>4 | 1<br>1<br>3 | 1<br>1<br>3 | 1<br>1<br>1 | 1<br>1<br>1 | 1<br>1<br>1 | 1<br>1<br>1 | 2<br>2<br>2 | 2<br>2<br>2 | 5 | 5 |
| 68 | 1<br>0,5<br>0,25 | 1<br>4<br>5 | 2<br>5 | 1<br>1<br>3 | 1<br>1<br>4 | 1<br>2<br>3 | 1<br>2<br>5 | 1<br>1<br>1 | 1<br>4<br>5 | 5 | 5 | 1<br>4<br>5 | 1<br>5 | 1<br>1<br>3 | 1<br>3<br>4 | 5 | 1<br>1<br>1 | 1<br>1<br>1 | 1<br>1<br>1 | 1<br>3<br>5 | 5 | 5 | 5 |
| 78 | 1<br>0,5<br>0,25 | 1<br>5<br>5 | 2<br>4<br>5 | 1<br>1<br>1 | 1<br>1<br>1 | 1<br>1<br>1 | 1<br>1<br>1 | 1<br>1<br>1 | 1<br>1<br>1 | 5 | 5 | 1<br>5 | 1<br>3<br>5 | 1<br>1<br>1 | 1<br>1<br>3 | 1<br>1<br>1 | 1<br>3<br>4 | 1<br>1<br>1 | 1<br>1<br>1 | 2<br>5 | 3<br>5 | 5 | 5 |
| 80 | 1<br>0,5<br>0,25 | 1<br>5 | 2<br>3<br>5 | 1<br>1<br>2 | 1<br>1<br>3 | 1<br>1<br>1 | 1<br>1<br>1 | 1<br>1<br>2 | 1<br>1<br>1 | 5 | 5 | 2<br>5 | 3<br>3<br>5 | 1<br>2<br>2 | 1<br>2<br>2 | 1<br>1<br>2 | 1<br>1<br>1 | 1<br>1<br>1 | 1<br>1<br>2 | 3<br>5 | 4<br>5 | 5 | 5 |
| 89 | 1<br>0,5<br>0,25 | 1<br>5 | 2<br>5 | 1<br>1<br>1 | 1<br>1<br>1 | 1<br>1<br>1 | 1<br>1<br>1 | 1<br>1<br>1 | 1<br>1<br>1 | 5 | 5 | 2<br>5 | 3<br>3<br>5 | 1<br>1<br>1 | 1<br>1<br>2 | 1<br>1<br>1 | 1<br>1<br>1 | 1<br>1<br>1 | 1<br>1<br>1 | 3<br>5 | 4<br>5 | 5 | 5 |
| 112 | 1<br>0,5<br>0,25 | 2<br>5 | 3<br>5 | 1<br>1<br>3 | 1<br>1<br>4 | 1<br>2<br>4 | 1<br>1<br>2 | 1<br>1<br>1 | 1<br>1<br>4 | 5 | 5 | 3<br>5 | 3<br>4<br>5 | 1<br>2<br>3 | 1<br>3<br>4 | 1<br>1<br>3 | 1<br>1<br>3 | 1<br>2<br>4 | 1<br>2<br>3 | 4<br>5 | 2<br>4<br>5 | 5 | 5 |

Tabelle 6: Selektive herbizide Wirkung der erfindungsgemäßen Verbindungen in Paddy-Reis

| Verbindung Nr. | kg Wirkstoff a.i./ha | Wirkungsgrad % REIS | ECHINOCHLOA CRUS GALLI |
|---|---|---|---|
| 78 | 3 | 32 | 100 |
| | 2 | 5 | 100 |
| | 1 | 0 | 100 |
| | 0,5 | 0 | 95 |
| 80 | 3 | 11 | 100 |
| | 2 | 4 | 100 |
| | 1 | 0 | 80 |
| | 0,5 | 0 | 66 |
| 89 | 3 | 28 | 100 |
| | 2 | 8 | 100 |
| | 1 | 0 | 100 |
| | 0,5 | 0 | 100 |
| B (bekannt) | 3 | 100 | 100 |
| | 2 | 100 | 100 |
| | 1 | 100 | 100 |
| | 0,5 | 100 | 100 |
| C (bekannt) | 3 | 10 | 100 |
| | 2 | 5 | 100 |
| | 1 | 0 | 65 |
| | 0,5 | 0 | 61 |
| D (bekannt) | 3 | 100 | 100 |
| | 2 | 100 | 100 |
| | 1 | 100 | 100 |
| | 0,5 | 100 | 96 |
| E (bekannt) | 3 | 0 | 100 |
| | 2 | 0 | 100 |
| | 1 | 0 | 93 |
| | 0,5 | 0 | 82 |

Patentansprüche:

1. Verwendung von Acyl-s-triazinen der allgemeinen Formel

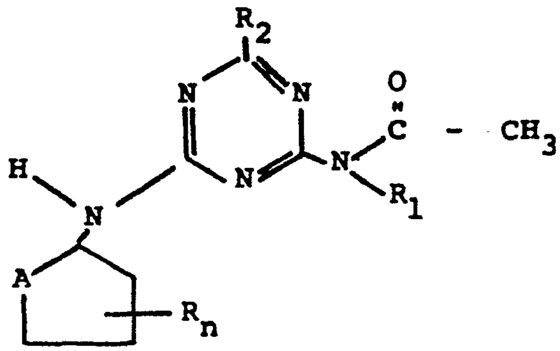

in der

$R_2$ Halogen, Alkoxy- oder Alkthio

$R_1$ einen gerad- oder verzweigtkettigen Alkyl-, Alkoxy-, Alkoxyalkyl- oder Alkylol-Rest,

R einen Rest aus der Gruppe der Alkyle mit ein bis drei C-Atomen und

A = $-CH_2-$ oder $-CH_2-CH_2-$ bedeuten und

n jeweils O oder 1, 2 oder 3

sein kann und wobei die Substituenten R an den Cycloalkylringen gleich oder verschieden sein können, in geeigneter Applikationsform zur selektiven Behandlung von Nutzpflanzen mit breitem Wirkungsspektrum gegen Unkräuter und Schadgräser

vor und/oder nach dem Auflaufen der Saat.

2. Verwendung von Mischungen von zwei oder mehreren Wirkstoffen nach Anspruch 1, bei denen sich die Einzelwirkstoffe durch unterschiedliche Substituenten $R_1$ und/oder R und/oder von A und/oder die Werte von n voneinander unterscheiden.

3. Verwendung eines oder mehrerer der Wirkstoffe nach Anspruch 1 oder 2 in Mengen von je 0,1 bis 10 kg/ha, vorzugsweise von 0,5 bis 2,5 kg/ha.

4. Verwendung eines oder mehrerer der Wirkstoffe nach Anspruch 1 oder 2 und 3,
dadurch gekennzeichnet,
daß man die jeweilige Applikationsform durch Zumischen von Hilfsmitteln aus der Gruppe der Träger-, Verdünnungs-, Lösungs-, Netz-, Haft-, Dispergier- und Emulgiermittel gewinnt.

5. Verwendung eines oder mehrerer der Wirkstoffe nach Anspruch 1 und einem oder mehreren der Ansprüche 2 bis 4,
gekennzeichnet durch
die Zumischung von Stoffen aus der Gruppe der Herbizide, Fungizide, Insektizide, Wachstumsregulatoren.

6. Verwendung eines oder mehrerer der Wirkstoffe nach Anspruch 1 und gegebenenfalls nach einem oder mehreren der Ansprüche 2 bis 5 als Zusatz zu Mineraldüngern.

Europäisches Patentamt

# EUROPÄISCHER RECHERCHENBERICHT

0166896 Nummer der Anmeldung

EP 85 10 4953

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | DE-B-1 271 452 (J.R. GEIGY)<br>--- | | A 01 N 43/70<br>C 07 D 251/50<br>C 07 D 251/52 |
| A | DE-B-1 445 055 (J.R. GEIGY)<br>--- | | |
| A | US-A-3 786 053 (T. CHAPMAN u.a.)<br>--- | | |
| A,D | CENTRAL PATENTS INDEX, Basic Abstracts, Journal, Section C: Agoc, Week T 27, 1972, Nr. 44009T, Derwert, London, GB; & JP - A - 72 23 436 (NIPPON KAYAKU K.K.) 30-06-1972<br>----- | | |
| | | | **RECHERCHIERTE SACHGEBIETE (Int Cl.4)** |
| | | | A 01 N<br>C 07 D |

Der vorliegende Recherchenbericht wurde für alle Patentansprüche erstellt.

| Recherchenort | Abschlußdatum der Recherche | Prüfer |
|---|---|---|
| DEN HAAG | 16-09-1985 | DECORTE D. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82